# EUROPEAN PATENT APPLICATION

(11) **EP 2 179 767 A1**
(43) Date of publication of application: **28.04.2010**
(21) Application number: 08475010.8
(22) Date of filing: 28.11.2008
(51) Int. Cl.: A61N 5/06, A61N 5/067, F21L 4/00

(54) **The nasal applicator for intranasal blood irradiation with laser**

(30) Priority: 21.10.2008 SK 1222008 U
(71) Applicant: Yalong Trade, s.r.o., 050 01 Revúca (SK)
(72) Inventor: Kokos, Frantisek, 050 01 Revúca (SK); Jurinyi, Ludovit, 059 39 Nizna Sunava (SK)
(74) Representative: Rzymanova, Kamila

(57) **Abstract**

The technical solution is related to a nasal applicator for intranasal blood irradiation with laser, comprising a semi-conductor laser instrument body (1), provided from outside with nasal clips (3) and a nasal adapter (5), connected together by means of a laser instrument interface (2), provided on both sides with hollow ends, wherein the light guide (4) passes through the centre of the laser instrument interface (2) and of the nasal adapter (5) and it ends in the opening of the nasal adapter (5).

## Description

### Technology area

The technical solution belongs to the area of radiation therapy and it pertains to the intranasal laser light application to irradiate the blood.

### Current state of technology

The blood irradiation with laser light represents a new healing method by which the laser light gets directly into the circulating blood. The basic knowledge of this method was that it influenced the rheological properties of blood. By microscopic blood examination it is possible to see the accumulations of blood platelets which reduce the blood oxidation. Following to the defined blood irradiation with laser light these accumulations are ripped apart, making the supply of blood with oxygen easier. Many studies report the positive effects on the blood and immunity system, as well as on almost all organs.

The area of treatment with laser light is very wide in consideration of various wave lengths of the light (red light with higher wave length, green and blue ones with lower wave length), applied either individually or in combination with other therapies. The targeted effects can be achieved by means of defined wave lengths. This is being a subject of long years of research and is reflected in the numerous representations of patent protections.

One of the known methods of laser light use is the transfusion therapy method, which is based on the plasma filtration method He-Ne, by which a certain quantity of blood is taken from the vein, it is treated against coagulation, irradiated with ultraviolet light and returned back to the vessel by means of re-transfusion. This method has positive effects' however the disadvantage thereof consists in working with patient's blood which can cause infection states. Another disadvantage is that only certain quantity of blood can be treated. The price of this therapy is high and it requires certain environment to be realized in.

Another known method is the method of intravasal laser irradiation by which it is not necessary to take blood, nor add any anti-coagulation agents. For such intravenous treatment it is necessary to introduce a cannula into the suitable vein of the elbow or forearm during the treatment procedure. This should be a thickest vein possible in order to get the major blood passage possible. The intravenous application is known (M.H.Weber,Th. Fussgänger-May, T.Wolf: Intravasal blood irradiation with laser - presentation of a new curative method), by which the laser resource is located in the apparatus and the laser light passes through a 2 m long light guide which is introduced into the cannula, located in the vein. The disadvantage of this method is that the cannula directly impacts the blood circulation. This damages the blood vessels and can easily cause infections. This therapy of intravascular laser irradiation is economically demanding and it can only be applied to a smaller group of people.

The disadvantages of these methods are eliminated by the prospective technical solution, based on the theoretical blood rheology principles, rules of body functioning and laser biology theory. According to the presented technical solution, the nasal cavity irradiation with laser (laser light) with wave length 650 nm and low intensity can be realized by means of nasal applicator. The objective of our protection is the applicator by which the laser light is applied into the blood circulation system without using cannulas or other interference with the blood system.

### Principle of technical solution

The principle of this technical solution consists in the nasal applicator for intranasal blood irradiation with laser, composed of the semi-conductor laser instrument body, provided form the outside with nasal clips and nasal adapter, connected together with an advantage with the laser instrument interface, equipped on both sides with the hollow stubs, whereby a light guide passes through the centre of both the laser instrument interface and nasal adapter, ending in the nasal adapter opening.

The principle of this technical solution also is that the laser radiation source is located with an advantage in the body of semi-conductor laser instrument, alternatively outside of semi-conductor laser instrument body, whereby the light guide is led from the source to the nasal adapter opening.

Both nasal adapter and interface are removable. The nasal adapter with the advantage contains the opening-free cover to protect the laser instrument interface.

Many researchers try to understand the principles of low-energy irradiation; they investigate various aspects in the areas like photon bio-physics, anatomy, physiology, biochemistry, immunology, nuclear medicine, imaging, molecular biology and others, but the issue is not so clear yet. Currently, there are three generally accepted theories (taken over with the permit of the authors from a Clinical study of the results of therapy by SLT semi-conductor therapeutic instrument by means of nasal cavity irradiation through Single Photon Emission Computed Tomography, Shaowei Jia, MD,PhD; Yu Shi, MD; Zhou Gao, MD, Beijing, University Shenzhen Hospital):
1. The energy released from the laser is certain type of stimulation. This stimulation effects locally the tissues and/or multi-sensors on the vascular wall and excites them as well as it effects the neuro-muscular fibres, free ceptors and blood corpuscles /Pacinis corpsules, Krause and bulb/, receptors, follicles, etc. The sensors receiving the laser stimulation are presumably the dissociative nerve twigs besides the micro-artery. When the stimulating signals enter the brain, they start to be processed by the brain and distributed to the relevant brain parts, helping this way the synthesis of brain encephalic nervous nucleic acids and DNA replication, as well as they regulate the functioning of enzymes, increase the brain perfusion with blood, stimulate the function of neural cells, improve the alimentation thereof, help the substitution, repair and regeneration of damaged cells and eliminate the pathological changes and processes.
2. The neural-internal secretion system is stimulated and adjusted by means of transduction and released laser energy, including the insulin, sex hormones (testosterone, oestradiol, prolane B /luteinizing hormone/, hormone stimulating the follicles), PRL, SOD, thyroid hormones, T-group of lymphocytes, immunoglobuline, alexine and others. This regulation has the characteristics of integration and double-tropism.
3. The traditional Chinese medicine believes that the head is composer of the "Yang" energy.
   "Du Mai" consists of the energy "Yang Mai",
   "Ren Mai" consists of the energy "Yin Mai".
   "Du Mai" and "Ren Mai" meet in the face..

The nose is situated in the head centre and the meridians pass through it, like
"Du Mai",
"Shou Zu Yang Ming Jing"
"Zu Tai Yang Jing".

Therefore, the laser stimulation in the nose opens the "Jing Luo" (nervous network) and increases the blood circulation "Qi" (energy or life power, stamina), due to what the healing takes place of many diseases.

The positive results and advantages of the semi-conductor laser therapy by means of nasal applicator according to this solution consist in the:
- simple and easy use of nasal cavity
- safe hygiene, since every patient ha his/her own nasal adapter
- nasal adapter is small, easy to carry
- possibility to use it in domestic environment but also in hospitals
- high quality nasal applicator
- low cost treatment

Besides the specific effects, the blood irradiation by means of nasal applicator in general takes effect in a significant improvement of overall performance, improved sleeping, positive effects on the mood and reduction of drugs consumption.

### Overview of the images on the drawings

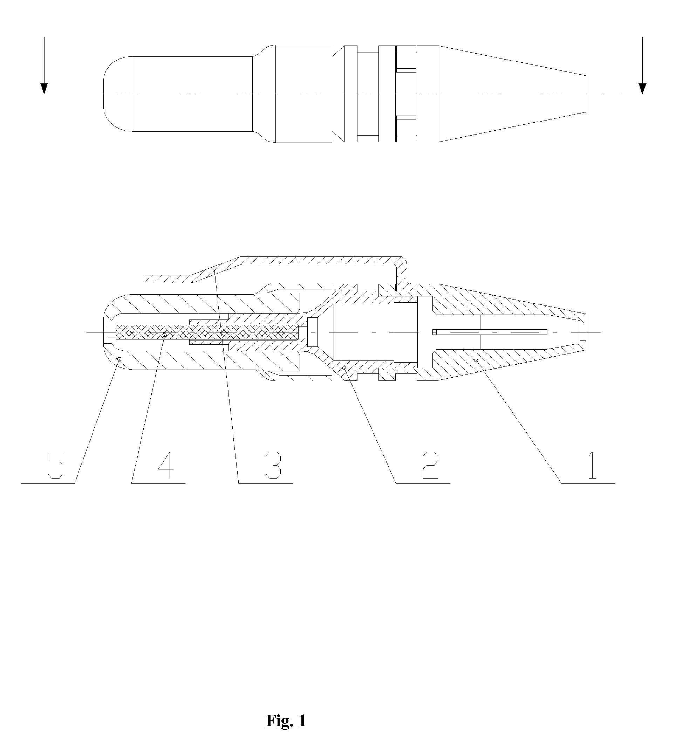

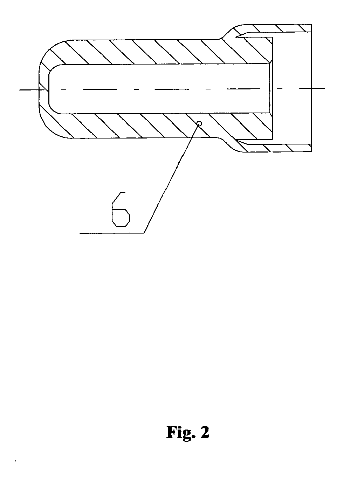

Figure 1 shows the example of the nasal applicator for intranasal blood irradiation with laser, with nasal adapter and Figure 2 shows the protection cover of the laser instrument interface.

### Examples of realization

### Example 1

An example of nasal applicator for the intranasal blood radiation with laser, shown on Fig. 1 consists of the body 1 of the semi conductor laser instrument, equipped from outside with a nasal clip 3 and nasal adapter 5, connected with an advantage by means of the interface 2 of the laser instrument, provided on both sides with hollow stubs, whereby a light guide 4 passes through the centre of the laser equipment interface 2 and nasal adapter 5 ending in the opening of the nasal adapter 5.

The laser radiation source is located in the body 1 of the semi-conductor laser instrument. Both nasal adapter 5 and interface 2 of the laser instrument are removable. The nasal adapter 5 can be replaced with the opening free cover 6 for the protection of the interface 2 of laser instrument (Fig. 2)

The nasal adapter 5 is inserted into the nasal cavity and secured by means of nasal clips 2 against falling out. At the beginning the irradiation with laser light is applied once a day for 5 minutes with the dose of 3mW and the time is extended step-by-step to 30 minutes; than the dose is changed to 4mW. The serious states are to be irradiated 2x a day for 30 minutes at 5mW; after one month the irradiation is reduced to 1x a day.

### Example 2

The nasal applicator for intranasal blood irradiation with laser is similar to the example 1, however the source of laser radiation is located outside the body 1 of the semi-conductor laser instrument, whereby the light guide 4 is led from the source to the nasal adapter opening 5.

### Example 3

The nasal applicator for intranasal blood irradiation with laser was used for transcutaneous irradiation of the surface of skin or subcutaneous layers by full contact with skin, whereas this wave length enters quite deep into the tissues and it is in accordance with the theory and practice of combination of blood irradiation with local irradiation. The laser source output is low, but it is sufficient to achieve the healing effect.

### Industrial utility

The nasal applicator in this solution can be used in practical medicine to heal various diseases, like diabetes mellitus, liver disorder, immunity system disorders and a research has started of usability of this instrument and verification thereof in the treatment of oncology and other illnesses.

### List of reference marks

1 Semi conductor laser instrument body
2 Interface
3 Nose clips
4 Light guide
5 Nasal adapter
6 Opening free cover

## Claims

1. The nasal applicator for intranasal blood irradiation with laser **characterized by** the fact of consisting of the semi conductor laser instrument body (1) provided from outside with the nasal clips (3) and of the nasal adapter (5), connected together with an advantage by means of the interface (2) of the laser instrument, provided on both sides by hollow stubs, whereby the light guide (4) passes through the centre of the laser instrument interface (2) and it ends in the opening of the nasal adapter (5).

2. The nasal applicator for intranasal blood irradiation with laser in accordance with the claim 1, **characterized by** the fact that the source of laser radiation is located with an advantage in the semi conductor laser instrument body (1), alternatively outside the body (1) of the semi conductor laser instrument, whereas the light guide (4) is led from the source to the opening of nasal adapter (5).

3. The nasal applicator for the intranasal blood irradiation with laser in accordance with the claim 1 **characterized by** the act that both nasal adapter (5) and interface (2) are removable.

4. The nasal applicator for intranasal blood irradiation with laser in accordance with the claim 1 **characterized by** the fact that the nasal adapter (5) with the advantage contains the opening free cover (6) for the protection of the laser instrument interface (2).
